# NEW EUROPEAN PATENT SPECIFICATION

(11) **EP 3 914 605 B2**
(45) Date of publication and mention of the opposition decision: **29.04.2026**
(45) Mention of the grant of the patent: 07.12.2022
(21) Application number: 20700941.6
(22) Date of filing: 23.01.2020
(51) Int. Cl.: C07K 1/30, C07K 1/04

(54) **PEPTIDE PRECIPITATION METHOD**
PEPTIDFÄLLUNGSVERFAHREN
MÉTHODE DE PRÉCIPITATION DES PEPTIDES

(30) Priority: 24.01.2019 EP 19153416
(43) Date of publication of application: 01.12.2021
(73) Proprietor: DSM IP Assets B.V., 6221 BE Maastricht (NL)
(72) Inventor: HEIDL, Marc, 4303 Kaiseraugst (CH); SCHAERER, Thomas, 4303 Kaiseraugst (CH)
(74) Representative: Kraus & Lederer PartGmbB
(86) International application number: PCT/EP2020/051573
(87) International publication number: WO 2020/152246

(56) References cited:
- WO-A1-2005/063793
- US-A1- 2005 165 215
- "Fmoc solid phase peptide synthesis : a practical approach", 16 December 1999, OXFORD UNIVERSITY PRESS, GB, ISBN: 978-0-19-963724-9, article C CHAN W ET AL: "Chapter 3, Section 10: TFA-mediated cleavage", pages: 64 - 76, XP055598795

## Description

Methods for the synthesis of peptides are provided. The methods include a specific precipitation step which facilitates the filtration of the peptide.

Peptides, both in solution and on solid phase, are generally synthesized from the C to N terminus by condensation of the carboxyl group of one amino acid to the amino group of another. However, peptides and amino acids from which peptides are synthesized tend to have reactive side chain functional groups. Thus, when synthesizing a peptide, it is important to assure that the respective amino group selectively reacts with the carboxyl group without any side reactions at such side chain functional groups as this could seriously impair yield or even ruin the product being synthesized from a practical perspective. To minimize such side reactions, it is therefore conventional practice to appropriately mask reactive side groups and terminal ends of reactants to help ensure that the desired reaction occurs.

As the respective amino-protecting groups (temporary protecting groups) are removed several times during the synthesis their removal must accordingly be done in mild conditions. In the meantime, reactive side chain groups of the amino acid and peptide reactants, including the resin-bound peptide material as well as the additional material to be added to the growing chain, typically remain masked with side chain protecting groups throughout the synthesis (permanent protecting groups) and are only removed in the last step of the synthetic process.

Well-known temporary protecting groups are the tert-Butyloxycarbonyl (Boc) and the 9-fluorenylmethoxycarbonyl (Fmoc) groups, which are either cleaved by weak acids or weak bases.

Cleavage of the permanent protecting groups as well as cleavage from the resin is however generally affected by strong acids or bases, optionally in the presence of a scavenger and a solvent. After the cleavage, the peptide has to be recovered from the cleavage solution.

Typical methods to recover peptides involve the use of acid/salt chemistry. For example, the peptide can be precipitated in aqueous salt (such as sodium chloride), and the solids can then be collected (for example, by vacuum filtration), washed, and dried. However, such methods present problems for commercial scale production, including high impurity levels. Other recovery methods involve precipitation of the peptide by the addition of a solvent such as in particular an ether solvent followed by collecting the precipitated peptide by filtration. Precipitation with an ether solvent, however, often leads to peptide precipitates which clog the filtration apparatus (for example, when precipitate is too fine) and/ or are sticky/tacky and thus also complicate or even prevent filtration (for example, when precipitate is gelatinous). International patent application WO 2005/063793 discloses that peptide precipitation by alcohol improves the filterability.

For large-scale production of peptides such issues can thus greatly impact the feasibility of the peptide synthesis scheme. Consequently, there is a continuing need for peptide synthesis processes capable of producing peptide materials of commercial interest in large batch quantities. Recovery of peptide material after synthesis, for example, by precipitation and subsequent filtration, is one aspect of the synthesis in which further improvement is needed as conventional methodologies often still require extensive filtration times which in turn significantly impacts production costs.

Surprisingly it has now been found that peptide precipitates prepared according to the methods according to the present invention can be filtered much faster than the ones precipitated according to standard methods in the art. Accordingly the methods according to the present invention can advantageously be used to provide improved commercial scale processes for synthesis and recovery of peptides.

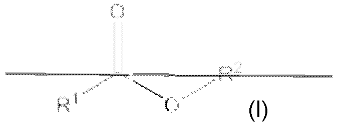

Thus, in a first embodiment the present invention relates to a method of obtaining a fully deprotected peptide, said method comprising the consecutive steps of
a.) synthesizing a peptide intermediate having at least one acid labile protecting group
b.) removing said protecting group(s) using a cleavage reagent consisting essentially of at least one acid, dodecanethiol, and toluene to form a cleaved peptide solution (I), followed by
c.) providing a solvent which is an ester solvent of formula (I) 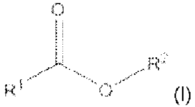 wherein R¹ and R² are independently of each other a linear or branched C₁-C₁₀ alkyl group or C₃-C₆ cycloalkyl group to the cleaved peptide solution (I) to form a cleaved peptide solution (II), followed by
d.) providing a precipitating agent selected from the group consisting of at least one ether solvent and at least one hydrocarbon solvent as well as mixtures thereof in an amount sufficient to precipitate the peptide from the cleaved peptide solution (II) and
e.) collecting the peptide.

In another aspect, the invention provides a method for precipitating a peptide comprising the consecutive steps of: (a) providing a composition consisting essentially of a peptide intermediate having at least one acid labile protecting group and a cleavage reagent consisting essentially of at least one acid, dodecanethiol, and toluene followed by (b) providing a solvent which is an ester solvent of formula (I) as defined above to the composition to obtain a cleaved peptide solution (II), followed by (c) providing a precipitating agent selected from the group consisting of an ether solvent and a hydrocarbon solvent as well as mixtures thereof to the cleaved peptide solution (II) followed by d.) collecting the peptide.

The term 'consisting essentially of' as used according to the present invention means that the total amount of the listed ingredients ideally sums upto 100 wt.-%. It is however not excluded that small amounts of impurities or additives may be present, with the proviso that the total amount of such impurities or additives is preferably less than 3 wt.-%, more preferably less than 2 wt.-%, most preferably less than 1 wt.-%.

In step b.) the addition of the organic solvent which is an ester solvent of formula (I) may result in an initial precipitation of the peptide. The amount of the solvent is however adjusted such, that in case of such a precipitation the peptide resolubilizes again to form a cleaved peptide solution. The amount of the organic solvent can easily be adjusted by a person skilled in the art and may slightly vary depending on the peptide sequence and the temperature applied in the dilution/ resolubilisation step b.).

Preferably step b.) is carried out at ambient temperature, i.e. at a temperature ranging from 18 to 25°C.

The incorporation of step b.) into the isolation step of the cleaved peptide provides dramatically reduced filtration times as compared to prior isolation techniques that omit the dilution/ resolubilization step as illustrated in the examples.

The term 'peptide intermediate having at least one acid labile protecting group' as used herein refers to a peptide comprising at least one, acid labile protecting group. The peptide intermediates according to the invention can accordingly be fully protected, i.e. all side chain reactive groups as well as the appropriate terminal amino acids include acid labile protecting groups (including N as well as C terminal protecting groups). In other aspects, the invention can however also be utilized to recover peptides wherein only all side chain reactive groups or only the terminal amino acids include acid labile protecting groups. In case of solid phase synthesis, the resin itself could also be acid labile and thus considered an acid labile protecting group. Preferably, the peptide intermediates according to the present invention however comprise at least on acid labile side chain protecting group.

An acid labile (side chain) protecting group refers to a chemical moiety coupled to the peptide such as preferably to the side chain (i.e. the R group in the general amino acid formula H₂N-C(R)(H)-COOH) of an amino acid that helps prevent undesired side reactions, such as e.g. prevents a portion of the side chain from reacting with chemicals used in steps of peptide synthesis, processing, and the like, and which can be removed with an acid, optionally in the presence of a scavenger. The choice of a suitable acid labile (side chain) protecting group can depend upon various factors, for example, the type of synthesis performed, processing to which the peptide will be subjected, and the desired intermediate product or final product. The acid labile (side chain) protecting group also depends upon the nature of the amino acid itself. Generally, an acid labile (side chain) protecting group is chosen that is not removed during deprotection of the α-amino groups during synthesis. Therefore, the α-amino protecting group and the side chain protecting group are typically not the same.

Examples of suitable acid labile (side chain) protecting groups according to the present invention include tert butyl (tBu), triphenylmethyl (trityl, trt), dimethoxytrityl (DMT), 2-chlorotrityl (2-CITrt), 1-cyclopropyl-1-methylethyl (Dmcp), tetrahydropyranyl, t-butoxycarbonyl (BOC), methoxytrimethylbenzene sulfonyl- (Mtr-), 2,2,5,7,8-pentamethyl-chroman-6-sulfonyl- (Pmc-), 2,2,4,6,7-pentamethyl-dihydrobenzofuran-5-sulfonyl- (Pbf-), adamantyloxycarbonyl, xanthyl (Xan), benzyl, 3-ethyl 3-pentyl, 5butyl 5-nonyl and t-butyl ester, benzyloxycarbonyl (Z), 2-chlorobenzyloxycarbonyl (2-CI-Z) and t-amyloxycarbonyl (Aoc) without being limited thereto.

Preferred acid labile side chain protecting groups in all embodiments according to the present invention include the t-Bu group for tyrosine, threonine, serine and aspartic acid amino acid residues; the trt group for histidine, glutamine, and asparagine amino acid residues; and the Boc group for lysine, DAB and tryptophan amino acid residues and the Mtr-, Pmc-, Bis-Boc or Pbf-group for arginine amino acid residues.

Suitable acids in the cleavage reagents are all acids which are able to fully deprotect the acid labile side chain protecting groups. The selection of an appropriate acid depends on the respective acid labile protecting groups used and can be easily selected by a person skilled in the art. Exemplary acids include carboxylic acids such as formic acid, acetic acid and trifluoro acetic acid, sulfonic acids such as methane sulfonic acid (MSA), trifluoromethane sulfonic acid (triflic acid), benzene sulfonic acid and p-toluene sulfonic acid, hydrogen bromide, trimethylsilyl trifluoromethanesulfonate (TMSOTf), trimethylsilyl bromid (TMSBr) as well as mixtures thereof. In all embodiments of the present invention the use of acetic acid, trifluoro acetic acid and methanesulfonic acid as well as mixtures thereof as acid in step b.) is however preferred. Most preferred is the use of trifluoro acetic acid and/ or methane sulfonic acid.

The total amount of acid(s) in the cleavage reagent is preferably selected in the range from 0.1 to 1000 mol-equivalents relative to the number of acid labile protecting groups present in the peptide intermediate, more preferably from 10 to 500 mol-equivalents, even more preferably from 20 to 400 mol-equivalents, and most preferably from 25 to 250 mol-equivalents.

According to the disclosure, suitable scavengers are all scavengers commonly used in peptide synthesis such as phenols, e.g., anisol, phenol; trialkylsilanes (more preferably C1-4-trialkylsilanes), e.g., trimethylsilane, triethylsilane or triisopropylsilane; thiols e.g. aliphatic thiols (alkylthiols), e.g., ethanedithiol, dodecanethiol or aromatic thiols, e.g., thioanisole and thiophenol and water as well as mixtures thereof. According to the present invention, the scavenger is dodecanethiol.

The total amount of scavenger(s) in the cleavage reagent is preferably selected in the range from 0.1 to 500 mol-equivalents relative to the number of acid labile protecting groups present in the peptide intermediate, more preferably from 1 to 100 mol-equivalents, even more preferably from 2 to 50 mol-equivalents, and most preferably from 3 to 40 mol-equivalents.

The cleavage reagent according to the present invention mandatorily comprises a scavenger which is dodecanethiol.

According to the disclosure, suitable aprotic solvents include liquid aromatic hydrocarbons (i.e. aromatic hydrocarbons, which are liquid at ambient temperature (i.e. 20°C)) or C₁₋₆-halogenoalkanes, such as in particular C₁₋₆-fluoro and/ or chloroalkanes, e.g. chloroform, dichloromethane, dichloroethane, 1,1-difluoroethane as well as mixtures thereof.- According to the present invention, the aprotic solvent is toluene.

If present, the amount of the aprotic solvent in the cleavage reagent is selected such that the peptide to be deprotected is solubilized therein at a temperature selected in the range from 10°C to 40°C, preferably from 0°C to 30°C, even more preferably from 10 to 25°. Preferably, the amount of the aprotic solvent in the cleavage reagent is selected in the range from 0.25 to 10 ml/g of peptide intermediate, more preferably in the range from 0.5 to 5 ml/ g of peptide intermediate, most preferably in the range of 0.75 to 1.5 ml/g of peptide intermediate. If not stated otherwise, the term peptide intermediate always refers to the fully protected peptide.

The total amount of the cleavage reagent is suitably selected in the range of 0.5 to 50 ml/ g of peptide intermediate, more preferably in the range from 1 to 50 ml/ g of peptide intermediate, most preferably in the range of 2.5 to 20 ml/ g of peptide intermediate (wherein the term peptide intermediate refers to the fully protected peptide).

Suitable ester solvents of formula (I) in all embodiment according to the present invention include linear or branched C₁-C₁₀ alkyl acetates, propionates, iso-propionates, butyrates and the like. Preferred ester solvents according to the present invention are linear or branched C₁-C₄ alkyl acetates such as e.g. methyl acetate, ethyl acetate, propyl acetate, isopropyl acetate, n-butyl acetate and tert.-butyl acetate, linear or branched C₁-C₄ alkyl propionates such as e.g. methyl propionate, ethyl propionate, propyl propionate, n-butyl propionate, tert-butyl propionate as well as C₁-C₄ alkyl butyrates such as e.g. methyl butyrate, ethyl butyrate, propyl butyrate, isopropyl butyrate, and tert.butyl butyrate. In all embodiments of the present invention the use of ethyl acetate, ethyl propionate, propyl acetate and diisopropyl acetate as well as mixtures thereof is however preferred.

The total amount of the organic solvent which is an ester solvent of formula (I) may vary depending on the respective peptide intermediate and can easily be adjusted by a person skilled in the art. If the peptide intermediate first precipitates upon the adition of the organic solvent, the amount of the organic solvent is selected such that the peptide resolubilizes. Preferably, in all embodiments of the present invention, the amount of the organic solvent is selected in the range from 0.5 to 100 ml/ g of peptide intermediate, more preferably in the range from 1 to 50 ml/ g of peptide intermediate, most preferably in the range of 5 to 35 ml/ g of peptide intermediate (wherein the term peptide intermediate refers to the fully protected peptide). Further suitable ranges include 10 to 30 ml/ g peptide intermediate or 15 to 30 ml / g peptide intermediate.

Suitable ether solvents as precipitation agent in all embodiment according to the present invention include cyclopentyl methyl ether, dibutyl ether, diethyl ether, diisopropyl ether, ethyl tert-butyl ether, methyl tert-butyl ether as well as mixtures thereof. In all embodiments of the present invention the use of diethyl ether and methyl tert-butyl ether as well as mixtures thereof is preferred.

Suitable hydrocarbon solvents as precipitation according to the present invention include pentane, hexane, heptane and petrolether as well as mixtures thereof. In all embodiments of the present invention the use petrolether and hexane as well as mixtures thereof is preferred.

If a mixture of ether and hydrocarbon solvents is used as a precipitating agent, the total amount of the hydrocarbon solvent in the precipitating agent should not exceed 35 Vol.-% based on the ether solvent used. In a particular advantageous embodiment according to the present invention, however, only an ether solvent is used as precipitating agent as this leads to particular advantageous results.

The precipitating agent is provided to the cleaved peptide solution (II) in an amount sufficient to precipitate the peptide from solution at a temperature selected in the range from -10°C to 40°C, preferably from 0°C to 30°C, even more preferably from 10 to 25°C and can easily be adjusted by a person skilled in the art. Preferably, the amount of precipitating agent is chosen to provide improved purity of the peptide precipitate. In some embodiments, the precipitating agent is provided in an amount of at least about 0.25 volumes or in the range of 0.25 to 8 volumes per volume of the cleaved peptide solution (II). In alternative embodiments, the precipitating agent is provided in an amount in the range of 0.5 to 5 volumes per volume of the cleaved peptide solution (II).

A cleavage reagent which is not in accordance with the present invention consists essentially of an acid, most preferably of trifluoroacetic acid, most preferably of 95% trifluoro acetic acid with all the definitions and preferences as given herein.

According to the present invention the cleavage reagent consists essentially of at least one acid, most preferably of trifluoroacetic acid and/ or MSA, dodecanethiol, and toluene with all the definitions and preferences as given herein.

The precipitated may be collected by known methods to a person skilled in the art such as by filtration or centrifugation. Preferably the precipitate is collected by filtration, even more preferably by filtration with a frit (fritted glass).

The methods described herein are particularly suitable for improving aspects of scaled-up synthesis of peptides. In preferred embodiments, the inventive methods can provide such improvements as reduction in processing (synthesis) time, improvements in the yield of products as well as improvements in product purity.

The methods of the present invention can be used in connection with the synthesis of peptides of any suitable length and/or sequence. It will be understood that the peptides of the invention can be synthesized or prepared by techniques well known in the art such as by solid phase synthesis or in solution. See, for example, Creighton, 1983, Proteins: Structures and Molecular Principles, W.H. Freeman and Co., NY, or M. Bodanszky, Principles of Peptide Synthesis, 1984, Springer Science & Business Media, as well as references cited herein.

In an advantageous aspect according to the present invention, the methods according to the present invention are used to recover peptides that incorporate about 2 to about 20, or about 2 to about 15, or about 3 to 12 residues of one or more amino acids or about 3 to about 6 residues of one or more amino acids. Residues of one or more other monomeric, oligomeric, and/or polymeric constituents optionally can be incorporated into a peptide. Non-peptide bonds may also be present. These non-peptide bonds can be between amino acid residues, between an amino acid and a non-amino acid residue, or between two non-amino acid residues. These alternative non-peptide bonds can be formed by utilizing reactions well known to those in the art, and may include, but are not limited to, imino, ester, hydrazide, semicarbazide, azo bonds, and the like.

The amino acids from which the peptides are derived can be naturally occurring amino acid residues, non-natural amino acid residues, or combinations thereof. The twenty common naturally-occurring amino acid residues are as follows: A (Ala, alanine), R (Arg, arginine); N (Asn, asparagine); D (Asp, aspartic acid); C (Cys, cysteine) Q (Gln, glutamine), E (Glu, glutamic acid); G (Gly, glycine); H (His, histidine); I (Ile, isoleucine); L (Leu, leucine); K (Lys, lysine); M (Met, methionine); F (Phe, phenylalanine); P (Pro, proline); S (Ser, serine); T (Thr, threonine); W (Trp, tryptophan); Y (Tyr, tyrosine); and V (Val, valine). Naturally occurring rare amino acids are also contemplated and include, for example, selenocysteine and pyrrolysine.

Non-natural amino acids include organic compounds having a similar structure and reactivity to that of naturally-occurring amino acids and include, for example, D-amino acids, beta-amino acids, omega-amino acids (such as 3-aminopropionic acid, 2,3-diaminopropionic acid, 2,4-diaminobutyric acid (DAB), 4-aminobutyric acid, and the like), gamma amino acids, cyclic amino acid analogs, propargylglycine derivatives, 2-amino-4-cyanobutyric acid derivatives without being limited thereto.

The peptides according to the present invention may be substituted at the terminal α-amino group with a lipophilic moiety connected to the peptide via an ester, amide, N-alkyl, N-alkenyl, sulfonyl, urethane, urea linkage. In particular, the peptide substituted with a lipophilic moiety refers to N-acyl derivatives thereof such as most in particular to the N-palmitoyl, N-Myristyl, N-Stearoyl, N-Benzoyl, N-Acetyl or N-Tetradecylaminocarbonyl derivatives thereof. Particularly preferred peptide derivatives in all embodiments of the present invention are N-Benzoyl or N-Tetradecylaminocarbonyl derivatives thereof.

The methods of the invention, which are directed to precipitating peptides, can be integrated into any peptide synthesis procedure, such as solid phase, liquid phase, hybrid synthesis, or recombinant synthesis.

When solid phase peptide synthesis (SPPS) is utilized, the synthesized peptide may be cleaved from the solid support (such as a resin) prior to utilization of the inventive methods described herein. Alternatively, the synthesized peptide may be cleaved from the solid support concomitantly with the removal of the protecting groups using the cleavage reagent according to the present invention.

In a particular preferred embodiment of the present invention the peptide intermediate is a peptide intermediate comprising at least one Mtr-, Pmc- or Pbf- side chain protected arginine and/ or at least one Boc side chain protected lysine and/ or 2,3-diaminopropionic acid and/ or 2,4-diaminobutyric acid (Dab) with all the definitions and preferences as given herein.

In a particular preferred embodiment of the present invention the peptide intermediate is a peptide intermediate (I) comprising at least one Mtr-, Pmc- or Pbf- side chain protected arginine and at least an amino acid carrying an electron rich aromatic side chain.

Preferred electron rich side chains in such peptide intermediates (I) are 'arylC₁-C₆ alkyl groups' and/ or 'heteroarylC₁-C₆ alkyl groups'.

The term "arylC₁-C₆ alkyl group" as used herein refers to a -C₁-C₆ alkyl-aryl group (i.e. to a C₁-C₆alkyl group which is substituted by an aryl group, i.e. the attachment point is the alkyl group), wherein the term "aryl" refers to an aromatic substituent containing 5 to 15 carbon atoms and containing a single aromatic ring or multiple aromatic rings which are fused together, directly linked or indirectly linked (such that the different aromatic rings are bound to a common group such as a methylene or ethylene group). Particularly advantageous aryl groups according to the present invention contain 6 to 12 carbon atoms containing a single aromatic ring or multiple aromatic rings which are fused together or directly linked. Most preferred aryl residues in all embodiments of the present invention are phenyl, naphthyl and biphenyl. Particularly advantageous arylC₁-C₆ alkyl groups in all embodiments of the present invention are arylC₁-C₂ alkyl groups such as in particular phenyl(m)ethyl or naphthyl(m)ethyl.

The term "heteroarylC₁-C₆ alkyl groups" refers to a -C₁-C₆ alkyl-heteroaryl (i.e.to a C₁-C₆alkyl group which is substituted by a heteroaryl group, i.e. the attachment point is the alkyl group), wherein the term "heteroaryl" refers to a 5-, 6- or 7-membered aromatic ring containing one or more heteroatoms, viz., N, O or S; these heteroaromatic rings may be fused to other aromatic systems. Particularly preferred heteroaromatic rings encompass imidazole, indole, pyridine and quinoline.

The aryl respectively heteroaryl residues may, independently of each other, be unsubstituted or substituted with one or more substituents. In all embodiments of the present invention, such substituents are preferably selected from halogen, hydroxy, nitro, cyano, C₁-C₆ alkyl, C₁-C₆ alkoxy and C₁-C₆ alkanoyloxy. More preferably in all embodiments of the present invention the aryl respectively heteroaryl residues are, independently of each other, unsubstituted or substituted with one or two substituents selected from the group consisting of F, Cl, hydroxy, cyano, C₁-C₃ alkyl, C₁-C₃ alkoxy and C₁-C₃ alkanoyloxy such as in particular unsubstituted or substituted with one or two substituents selected from the group consisting of F or hydroxy. Most preferably, in all embodiments of the present invention, the aryl and the heteroaryl residues are unsubstituted or substituted with F, hydroxy or nitro.

In all peptide intermediates (I) particular advantageous arylC₁-C₆ alkyl group are arylC₁-C₂ alkyl groups, which are preferably unsubstituted or substituted with one or two substituents selected from the group of fluoro (F), hydroxy (OH) or nitro (NO₂). Most preferred arylC₁-C₆ alkyl group in all embodiments of the present invention are phenyl(m)ethyl, 4-hydroxyphenyl(m)ethyl, 3-nitro-4-hydroxyphenyl(m)ethyl or naphthyl(m)ethyl such as in particular phenylmethyl, 4-hydroxyphenyl(m)ethyl, 3-nitro-4-hydroxyphenylmethyl or 1- or 2-naphthylmethyl.

In all peptide intermediate (I) most preferred heteroarylC₁-C₆ alkyl group are heteroarylC₁-C₂ alkyl groups such as (1H-indol-3-yl)(m)ethyl, (1H-imidazol-4-yl)(m)ethyl, (pyridin-2-yl)(m)ethyl, (pyridin-3-yl)(m)ethyl, (quinolin-2-yl)(m)ethyl and (quinolin-3-yl)(m)ethyl groups which are preferably unsubstituted or substituted with one or two substituents selected from the group of F (fluoro), hydroxy (OH) or nitro (NO₂). Most preferred in all embodiment of the present invention are (1H-indol-3-yl)(m)ethylene, or (1H-imidazol-4-yl)(m)ethylene.

In all peptide intermediates (I) the most preferred amino acids carrying an electron rich aromatic side chain are selected from the group of D/L-histidine, D/L-tryptophan, 6-fluoro-tryptophan, 5-hydroxy-tryptophan, D/L-phenylalanine, D/L-tyrosine, D/L-m-nitrotyrosine, D/L-O-ethyl-tyrosine, D/L-3-(2-naphthyl)-alanine. Particularly advantageous amino acids carrying an electron rich aromatic side chain are selected from the group of D/L-Histidine, D/L-Phenylalanine, D/L-tyrosine and D/L-tryptophan as well as O-C₁-C₆-alkylated D/L-tyrosine; m/p-Cl/Br/ID/L-phenylalanine; and 4/-5-/6-/7-OH/F/Cl/Br/methyl-D/L-tryptophan. Most preferred electron rich side chains in all embodiments of the present invention are the ones of D/L-histidine, D/L-tryptophan, D/L- tyrosine, D/L-tryptophan, 5-hydroxy D/L-tryptophan and 6-fluoro D/L-tryptophan. It is well understood that the term D/L encompasses the respective D-amino acids, L-amino acids as well as mixtures thereof.

In all embodiments of the present invention most preferably the peptide intermediate (I) comprising a Mtr-, Pmc- or Pbf- side chain protected arginine also contains at least an amino acid carrying an electron rich aromatic side chain selected from the group of D/L-histidine, D/L-tryptophan, tyrosine, D/L-6-fluoro-tryptophan and D/L-5-hydroxy-tryptophan..

Even more preferably, the peptide intermediate (I) is a di- to a hexapeptide, i.e. a dipeptide, a tripeptide, a tetrapeptide, a pentapeptide or a hexapeptide.

In a further preferred embodiment the Mtr-, Pmc- or Pbf- side chain protected arginine and the at least an amino acid carrying an electron rich aromatic side chainin the peptide intermediate (I) are seperated by at most 3 amino acids, preferably by at most two amino acids and more preferably by at most one amino acid. In all embodiments of the present invention however, the electron rich amino acid is most preferably directly adjacent to the Mtr-, Pmc- or Pbf- side chain protected arginine.

Even more preferably, in all embodiments of the present invention the peptide intermediate (I) is a peptide intermediate (II) comprising at least one Pbf-protected arginine moiety and at least one amino acid moiety selected from the group of tyrosine, histidine (preferably Trt protected) and/ or tryptophan which histidine, tyrosine respectively tryptophan moiety is preferably at most two, more preferably at most one, such as in particular directly adjacent to the protected arginine moiety. Particularly preferred tyrosine, histidine and/ or tryptophan amino acids are histidine, tyrosine, tryptophan, 5-hydroxytryptophane and 6-fluoro tryptophan as well as mixtures thereof. It is well understood, that as mentioned above, the Pbf-protected arginine moiety as well as the amino acid moieties can be L- or D- configurated or be mixtures of both configurations (within the peptide).

Particularly preferred peptide intermediates (I) respectively (II) according to the present invention are Boc-Trp-Arg(Pbf)-OH, Ac-Arg(Pbf)-His(Trt)-Phe-OH, TFA*H-Tyr(tBu)-Arg(Pbf)-Pro-OH, Boc-rac-6FTrp-Gly-Arg(Pbf)-Glu(OtBu)-OH, Boc-Tyr(Et)-Arg(Pbf)-Ala-Phe-OH, Ac-5-(OH)Trp-Ala-Arg(Pbf)-Ser(tBu)-Leu-Phe-OH, Boc-Arg(Mtr)-Tyr(tBu)-Phe-OH, Boc-2Nal-Leu-Arg(Pbf)-Phe-OH, Ac-Arg(Pbf)-Met-m(NO2)Tyr-Pro-OH and Bz-Gly-His(Trt)-D-Phe-Arg(Pbf)-D-Trp-N(Pr)2. Bz-Gly-His-D-Phe-Arg(Pbf)-D-Trp-NPr2, Bz-Gly-His(Trt)-D-Phe-Arg(Pbf)-D-Trp(Boc)-NPr2. The most preferred peptide intermediate (I) respectively (II) is Bz-Gly-His(Trt)-D-Phe-Arg(Pbf)-Trp-NPr₂.

In another particular preferred embodiment of the present invention the peptide intermediate is a peptide intermediate (III) comprising next to the at least one Boc side chain protected lysine and/ or 2,3-diaminopropionic acid and/ or 2,4-diaminobutyric acid (Dab) and/ or at least one valine or leucine or isoleucine. Even more preferably said peptide intermediates (III) are peptide intermediates (IV) consisting only of Boc side chain protected lysin or 2,4-diaminobutyric acid and valine.

Even more preferably, the peptide intermediate (III) and (IV) is a di- to a hexapeptide, i.e. a dipeptide, a tripeptide, a tetrapeptide, a pentapeptide or a hexapeptide.

Most preferred peptide intermediates (III) and (IV) according to the resent invention are TDAC-Dab(Boc)-Val-Dab(Boc)-OH, TDAC-Dab(Boc)-Val-Dab(Boc)-O-resin and Ac-Lys(Boc)-Val-Lys(Boc)-Val- Lys(Boc)-Val-OH.

The invention is further illustrated with reference to the following, non-limiting examples, in which all percentages are by weight based on total weight unless otherwise specified.

### Example

### Abbreviations

- Ac: acetyl
- Arg: arginine
- Boc: tert-butyloxycarbonyl
- Bz: benzoyl
- DCM: dichloromethane
- D-Phe: D-Phenylalanine
- EtOAc: ethyl acetate
- EtOPr: Ethyl propionate
- Et₂O: diethylether
- Gly: glycine
- iPropOAc: isopropyl acetate
- min: minute
- MSA: methanesulfonic acid
- MTBE: methyl tert-butyl ether
- Pbf: 2,2,4,6,7-pentamethyl-dihydrobenzofurane-5-sulfonyl
- PrOAc: propyl acetate
- TFA: trifluoroacetic acid
- Trp: tryptophane
- Trt: trityl
- Val: Valine

### Standard (reference) method:

The respective protected peptide (800 mg) as outlined in table 1 and 2 was placed in a flask. Afterwards the cleavage reagent (Ref 1: 0.8ml Toluene, 387 µl dodecanethiol, 245 µl MSA and 4.15 ml 100% TFA; Ref 2 and 3: 5.0 ml 95% TFA) was added to the peptide and the resulting reaction mixture was stirred for 1h to form the cleaved peptide solution (I).

Then, the cleaved peptide solution (I) was transferred onto 43ml MTBE resulting in the precipitation of the cleaved peptide. The resulting peptide suspension was stirred at ambient temperature for about 15 min. Afterwards the peptide suspension was transferred into a G4 pore size glass frit. The liquid level was marked and reduced pressure of 300mbar was applied to the frit. After the liquid level decreased to 2 cm (measured from the top of the frit), the filtration time was recorded until all liquid was removed from the frit.

### Method according to the invention:

The respective protected peptide (800 mg) was placed in a flask. Afterwards the cleavage reagent (I-1 to I-10: 0.8ml Toluene, 387 µl dodecanethiol, 245 µl methane sulfonic acid and 4.15 ml 100% TFA; I-11 and I-1: 5.0 ml 95% TFA) was added to the peptide and the resulting reaction mixture was stirred for 1h to form the cleaved peptide solution (I). Then, the cleaved peptide solution (I) was transferred onto an organic solvent as outlined in table 1 and 2 (which in case of I-5, I-6, I-7, I-11, I-12, I-13 resulted in an initial precipitation of the peptide, which was then solubilized again). The stirring was continued at ambient temperature (about 20°C) resulting in a cleaved peptide solution (II) (about 15 min). Then, the precipitation agent was added to the cleaved peptide solution (II) and the resulting peptide suspension was stirred for another 15 min. Afterwards the peptide suspension was transferred into a G4 pore size glass frit. The liquid level was marked and reduced pressure of 300mbar was applied to the frit. After the liquid level decreased to 2 cm (measured from the top of the frit), the filtration time was recorded until all liquid was removed from the frit.

**Table 1: Deprotection of Bz-Gly-His(Trt)-D-Phe-Arg(Pbf)-Trp-NPr₂**

| **#** | **R-1** | **I-1** | **I-2** | **I-3** | **I-4** | **I-5** | **I-6** | **I-7** | **I-8** | **I-9** | **I-10** |
|---|---|---|---|---|---|---|---|---|---|---|---|
| EtOAc¹ | | 21.5 | 21.5 | 21.5 | 21.5 | | | | | | 21.5 |
| EtOPr¹ | | | | | | 21.5 | | | | | |
| PrOAc¹ | | | | | | | 21.5 | | | | |
| iPropOAc¹ | | | | | | | | 21.5 | | | |
| DCM¹ (Reference example) | | | | | | | | | 10.8 | | |
| CHCl₃¹ (Reference example) | | | | | | | | | | 21.5 | |
| MTBE² | 43 | 21.5 | | | | 21.5 | 21.5 | 21.5 | 32.3 | 21.5 | |
| Et₂O² | | | 21.5 | | | | | | | | 13.3 |
| diisopropylether² | | | | 21.5 | | | | | | | |
| petrolether² | | | | | 21.5 | | | | | | |
| toluene² | | | | | | | | | | | 8.3 |
| Filtration time [min] | 80 | 7 | 2 | 2 | 34 | 4 | 5 | 22 | 63 | 1 | 1 |
| ¹organic solvent [ml] | | | | | | | | | | | |
| ²precipitation agent [ml] | | | | | | | | | | | |

**Table 2 (not according to the present invention): Deprotection of TDAC-Dab(Boc)-Val-Dab(Boc)-OH (R-2 & I-11 & I-12) respectively Ac-Lys(Boc)-Val-Lys(Boc)-Val-Lys(Boc)-Val-OH (R-3 and I-13)**

| **#** | **R-2** | **I-11** | **I-12** | **R-3** | **I-13** |
|---|---|---|---|---|---|
| EtOAc¹ | | 21.5 | 21.5 | | 21.5 |
| MTBE² | 43 | 21.5 | | | |
| i-propylether² | | | 21.5 | 43 | 21.5 |
| Filtration time [min] | 67 | 0.6 | 0.5 | 72 | 58 |
| ¹organic solvent [ml] | | | | | |
| ²precipitation agent [ml] | | | | | |

## Claims

1. A method of obtaining a fully deprotected peptide, said method comprising the consecutive steps of:
a) synthesizing a peptide intermediate having at least one acid labile protecting group
b) removing said protecting group(s) using a cleavage reagent consisting essentially of at least one acid, dodecanethiol, and toluene to form a cleaved peptide solution (I), followed by
c) providing an organic solvent which is an ester solvent of formula (I) 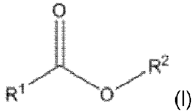 wherein R¹ and R² are independently of each other a linear or branched C₁-C₁₀ alkyl group or C₃-C₆ cycloalkyl group
to the cleaved peptide solution (I) to form a cleaved peptide solution (II), followed by
d) providing a precipitating agent selected from the group consisting of at least one ether solvent and at least one hydrocarbon solvent as well as mixtures thereof in an amount to precipitate the peptide from the cleaved peptide solution (II) and
e) collecting the precipitate.

2. The method according of claim 1, **characterized in that** the acid in step b) is selected from the group of formic acid, acetic acid, trifluoro acetic acid, a sulfonic acid, hydrogen bromide, trimethylsilyl trifluoromethanesulfonate, trimethylsilyl bromide as well as mixtures thereof, preferably from acetic acid, trifluoro acetic acid and methanesulfonic acid as well as mixtures thereof.

3. The method according to claim 1 or 2 **characterized in that** the amount of the acid is selected in the range from 0.1 to 1000 mol equivalents relative to the number of acid labile protecting groups present in the peptide intermediate, more preferably from 10 to 500 mol-equivalents, even more preferably from 20 to 400 mol-equivalents.

4. The method according to anyone of the preceding claims, **characterized in that** the amount of the scavenger is selected in the range from 0.1 to 500 mol equivalents relative to the number of acid labile protecting groups present in the peptide intermediate, more preferably from 1 to 100 mol-equivalents, even more preferably from 2 to 50 mol-equivalents.

5. The method according to anyone of the preceding claims, **characterized in that** the amount of the aprotic solvent is selected in the range from 0.25 to 10 ml/g of peptide intermediate, more preferably in the range from 0.5 to 5 ml/ g of peptide intermediate, most preferably in the range of 0.75 to 1.5 ml/g of peptide intermediate.

6. The method according to anyone of the preceding claims, **characterized in that** the amount of the cleavage reagent is selected in the range from 0.5 to 50 ml/ g of peptide intermediate, more preferably in the range from 1 to 50 ml/ g of peptide intermediate, most preferably in the range of 2.5 to 20 ml/ g of peptide intermediate.

7. The method according to anyone of the preceding claims, **characterized in that** the amount of the organic solvent in step c.) is selected in the range from 0.5 to 100 ml/g of peptide intermediate, more preferably in the range from 1 to 50 ml/ g of peptide intermediate, most preferably in the range of 5 to 35 ml/ g of peptide intermediate.

8. The method according to anyone of the preceding claims, **characterized in that** the ester solvent of formula (I) is selected from the group of ethyl acetate, ethyl propionate, propyl acetate and diisopropyl acetate as well as mixtures thereof.

9. The method according to anyone of the preceding claims, **characterized in that** the ether solvent in step d.) is selected from the group of cyclopentyl methyl ether, dibutyl ether, diethyl ether, diisopropyl ether, ethyl tert-butyl ether, methyl tert-butyl ether as well as mixtures thereof, preferably from diethyl ether and methyl tert-butyl ether as well as mixtures thereof.

10. The method according to anyone of the preceding claims, **characterized in that** the peptide incorporates 2 to 20, preferably 2 to 15, more preferably 3 to 12, most preferably 3 to 6 residues of one or more amino acids.

11. The method according to anyone of the preceding claims, **characterized in that** the peptide is substituted at the terminal α-amino group with a lipophilic moiety selected from the group of acetyl, benzoyl or tetradecylaminocarbonyl moieties.

12. The method according to anyone of the preceding claims, **characterized in that** the peptide intermediate comprises at least one one Mtr-, Pmc- or Pbf- side chain protected arginine and/ or at least one Boc side chain protected lysin, 2,3-diaminopropionic acid or 2,4-diaminobutyric acid.

13. The method according to anyone of the preceding claims, **characterized in that** the peptide intermediate is selected from the group of Bz-Gly-His(Trt)-D-Phe-Arg(Pbf)-DTrp-N(Pr)₂, Bz-Gly-His-D-Phe-Arg(Pbf)-D-Trp-N(Pr)₂, Bz-Gly-His-D-Phe-Arg(Boc)2-DTrp-N(Pr)₂, Bz-Gly-His(Trt)-D-Phe-Arg(Boc)2-D-Trp-N(Pr)₂, TDAC-Dab(Boc)-Val-Dab(Boc)-OH, TDAC-Dab(Boc)-Val-Dab(Boc)-O*^{t}*Bu, TDAC-Dab(Boc)-Val-Dab(Boc)-O-resin, Ac-Lys(Boc)-Val- Lys(Boc)-Val- Lys(Boc)-Val-OH.

## Patentansprüche

1. Verfahren zur Gewinnung eines vollständig entschützten Peptids, wobei das Verfahren die aufeinanderfolgenden Schritte umfasst:
a) Synthetisieren einer Peptidzwischenstufe mit wenigstens einer säurelabilen Schutzgruppe
b) Entfernen der Schutzgruppe(n) unter Verwendung eines Spaltungsreagens, das im Wesentlichen aus wenigstens einer Säure, Dodecanthiol und Toluol besteht, so dass eine Lösung (I) on gespaltenem Peptid gebildet wird, gefolgt von
c) Zugeben eines organischen Lösungsmittels, das ein Esterlösungsmittel der Formel (I) ist 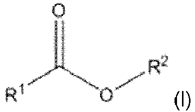
wobei R¹ und R² unabhängig voneinander für eine unverzweigte oder verzweigte C₁-C₁₀ -Alkylgruppe oder eine C₃-C₆-Cycloalkylgruppe stehen,
zu der Lösung (I) von gespaltenem Peptid, so dass eine Lösung (II) von gespaltenem Peptid gebildet wird, gefolgt von
d) Bereitstellen eines Fällungsmittels, ausgewählt aus der Gruppe bestehend aus wenigstens einem Etherlösungsmittel und wenigstens einem Kohlenwasserstofflösungsmittel sowie Gemischen davon in einer Menge, so dass das Peptid aus der Lösung (II) von gespaltenem Peptid gefällt wird, und
e) Sammeln des Niederschlags umfasst.

2. Verfahren nach Anspruch 1, **dadurch gekennzeichnet, dass** die Säure in Schritt b) ausgewählt ist aus der Gruppe Ameisensäure, Essigsäure, Trifluoressigsäure, einer Sulfonsäure, Bromwasserstoff, Trimethylsilyltrifluormethansulfonat, Trimethylsilylbromid sowie Gemischen davon, bevorzugt aus Essigsäure, Trifluoressigsäure und Methansulfonsäure sowie Gemischen davon.

3. Verfahren nach Anspruch 1 oder 2, **dadurch gekennzeichnet, dass** die Menge der Säure im Bereich von 0,1 bis 1000 Moläquivalenten relativ zur Anzahl säurelabilen Schutzgruppen, die in der der Peptidzwischenstufe vorliegen, bevorzugter von 10 bis 500 Moläquivalenten, noch bevorzugter von 20 bis 400 Moläquivalenten ausgewählt wird.

4. Verfahren nach einem der vorstehenden Ansprüche, **dadurch gekennzeichnet, dass** die Menge des Scavengers im Bereich von 0,1 bis 500 Moläquivalenten, relativ zur Anzahl säurelabilen Schutzgruppen, die in der der Peptidzwischenstufe vorliegen, bevorzugter von 1 bis 100 Moläquivalenten, noch bevorzugter von 2 bis 50 Moläquivalenten, ausgewählt wird.

5. Verfahren nach einem der vorstehenden Ansprüche, **dadurch gekennzeichnet, dass** die Menge des aprotischen Lösungsmittels im Bereich von 0,25 bis 10 ml/g Peptidzwischenstufe, bevorzugter im Bereich von 0,5 bis 5 ml/g Peptidzwischenstufe, am bevorzugtesten im Bereich von 0,75 bis 1,5 ml/g Peptidzwischenstufe ausgewählt wird.

6. Verfahren nach einem der vorstehenden Ansprüche, **dadurch gekennzeichnet, dass** die Menge des Spaltmittels im Bereich von 0,5 bis 50 ml/g Peptidzwischenstufe, bevorzugter im Bereich von 1 bis 50 ml/g Peptidzwischenstufe, am bevorzugtesten im Bereich von 2,5 bis 20 ml/g Peptidzwischenstufe ausgewählt wird.

7. Verfahren nach einem der vorstehenden Ansprüche, **dadurch gekennzeichnet, dass** die Menge des organischen Lösungsmittels in Schritt c) im Bereich von 0,5 bis 100 ml/g Peptidzwischenstufe, bevorzugter im Bereich von 1 bis 50 ml/g Peptidzwischenstufe, am bevorzugtesten im Bereich von 5 bis 35 ml/g Peptidzwischenstufe ausgewählt wird.

8. Verfahren nach einem der vorstehenden Ansprüche, **dadurch gekennzeichnet, dass** das Esterlösungsmittel der Formel (I) ausgewählt ist aus der Gruppe Essigsäureethylester, Propionsäureethylester, Essigsäurepropylester und Essigsäurediisopropylester sowie Gemische davon.

9. Verfahren nach einem der vorstehenden Ansprüche, **dadurch gekennzeichnet, dass** das Etherlösungsmittel in Schritt d.) ausgewählt ist aus der Gruppe Cyclopentylmethylether, Dibutylether, Diethylether, Diisopropylether, Ethyl-tert-butylether, Methyl-tert-butylether sowie Gemische davon, bevorzugt aus Diethylether und Methyl-tert-butylether sowie Gemische davon.

10. Verfahren nach einem der vorstehenden Ansprüche, **dadurch gekennzeichnet, dass** das Peptid 2 bis 20, bevorzugt 2 bis 15, bevorzugter 3 bis 12, am bevorzugtesten 3 bis 6 Reste einer oder mehrerer Aminosäuren enthält.

11. Verfahren nach einem der vorstehenden Ansprüche, **dadurch gekennzeichnet, dass** das Peptid an der endständigen α-Aminogruppe mit einer lipophilen Gruppierung ausgewählt aus der Gruppe Acetyl-, Benzoyl- oder Tetradecylaminocarbonyl-Einheiten substituiert ist.

12. Verfahren nach einem der vorstehenden Ansprüche, **dadurch gekennzeichnet, dass** das Peptidzwischenstufe wenigstens ein eine Mtr-, Pmc- oder Pbf-Seitenketten-geschütztes Arginin und/oder wenigstens ein Boc-Seitenketten-geschütztes Lysin, 2,3-Diaminopropionsäure oder 2,4-Diaminobuttersäure umfasst.

13. Verfahren nach einem der vorstehenden Ansprüche, **dadurch gekennzeichnet, dass** das Peptidzwischenstufe ausgewählt ist aus der Gruppe, bestehend aus Bz-Gly-His(Trt)-D-Phe-Arg(Pbf)-DTrp-N(Pr)₂, Bz-Gly-His-D-Phe-Arg(Pbf)-D-Trp-N(Pr)₂, Bz-Gly-His-D-Phe-Arg(Boc)2-DTrp-N(Pr)₂, Bz-Gly-His (Trt)-D-Phe-Arg(Boc)2-D-Trp-N(Pr)₂, TDAC-Dab(Boc)-Val-Dab(Boc)-OH, TDAC-Dab(Boc)-Val-Dab(Boc)-O*^{t}*Bu, TDAC-Dab(Boc)-Val-Dab(Boc)-O-resin, Ac-Lys(Boc)-Val-Lys(Boc)-Val- Lys (Boc)-Val-OH.

## Revendications

1. Procédé d'obtention d'un peptide entièrement déprotégé, ledit procédé comprenant les étapes consécutives suivantes :
a) la synthèse d'un peptide intermédiaire ayant au moins un groupe protecteur labile en milieu acide
b) l'élimination dudit ou desdits groupe(s) protecteur(s) en utilisant un réactif de clivage constitué essentiellement d'au moins un acide, de dodécanethiol et de toluène pour former une solution de peptide clivé (I), suivie par
c) la fourniture d'un solvant organique qui est un solvant ester de formule (I) 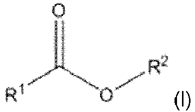
dans laquelle R¹ et R² sont indépendamment l'un de l'autre un groupe alkyle en C₁-C₁₀ linéaire ou ramifié ou un groupe cycloalkyle en C₃-C₆
à la solution (I) de peptide clivé pour former une solution (II) de peptide clivé, suivie par
d) la fourniture d'un agent de précipitation choisi dans le groupe constitué par au moins un solvant de type éther et au moins un solvant hydrocarboné ainsi que des mélanges correspondants en une quantité suffisante pour précipiter le peptide à partir de la solution (II) de peptide clivé et
e) la collecte du précipité.

2. Procédé selon la revendication 1, **caractérisé en ce que** l'acide dans l'étape b) est choisi parmi le groupe composé de l'acide formique, l'acide acétique, l'acide trifluoroacétique, un acide sulfonique, le bromure d'hydrogène, le trifluorométhanesulfonate de triméthylsilyle, le bromure de triméthylsilyle ainsi que des mélanges correspondants, préférence parmi l'acide acétique, l'acide trifluoroacétique et l'acide méthanesulfonique, ainsi que des mélanges correspondants.

3. Procédé selon la revendication 1 ou 2, **caractérisé en ce que** la quantité de l'acide est choisie dans la plage de 0,1 à 1000 équivalents molaires par rapport au nombre de groupes protecteurs labiles à l'acide présents dans le peptide intermédiaire, plus préférablement de 10 à 500 équivalents molaires, encore plus préférablement de 20 à 400 équivalents molaires.

4. Procédé selon l'une quelconque des revendications précédentes, **caractérisé en ce que** la quantité de l'agent de piégeage est choisie dans la plage de 0,1 à 500 équivalents molaires par rapport au nombre de groupes protecteurs labiles à l'acide présents dans le peptide intermédiaire, plus préférablement de 1 à 100 équivalents molaires, encore plus préférablement de 2 à 50 équivalents molaires.

5. Procédé selon l'une quelconque des revendications précédentes, **caractérisé en ce que** la quantité du solvant aprotique est choisie dans la plage de 0,25 à 10 ml/g de peptide intermédiaire, plus préférablement dans la plage de 0,5 à 5 ml/g de peptide intermédiaire, le plus préférablement dans la plage de 0,75 à 1,5 ml/g de peptide intermédiaire.

6. Procédé selon l'une quelconque des revendications précédentes, **caractérisé en ce que** la quantité du réactif de clivage est choisie dans la plage de 0,5 à 50 ml/g de peptide intermédiaire, plus préférablement dans la plage de 1 à 50 ml/g de peptide intermédiaire, le plus préférablement dans la plage de 2,5 à 20 ml/g de peptide intermédiaire.

7. Procédé selon l'une quelconque des revendications précédentes, **caractérisé en ce que** la quantité du solvant organique dans l'étape c.) est choisie dans la plage de 0,5 à 100 ml/g de peptide intermédiaire, plus préférablement dans la plage de 1 à 50 ml/g de peptide intermédiaire, le plus préférablement dans la plage de 5 à 35 ml/g de peptide intermédiaire.

8. Procédé selon l'une quelconque des revendications précédentes, **caractérisé en ce que** le solvant de type ester de formule (I) est choisi dans le groupe composé de l'acétate d'éthyle, le propionate d'éthyle, l'acétate de propyle et l'acétate de diisopropyle ainsi que des mélanges correspondants.

9. Procédé selon l'une quelconque des revendications précédentes, **caractérisé en ce que** le solvant de type éther dans l'étape d.) est choisi dans le groupe composé de l'éther méthylique de cyclopentyle, l'éther dibutylique, l'éther diéthylique, l'éther diisopropylique, l'éther tert-butylique d'éthyle, l'éther tert-butylique de méthyle ainsi que des mélanges correspondants, préférablement parmi l'éther diéthylique et l'éther tert-butylique de méthlye ainsi que des mélanges correspondants.

10. Procédé selon l'une quelconque des revendications précédentes, **caractérisé en ce que** le peptide incorpore 2 à 20, préférablement2 à 15, plus préférablement 3 à 12, le plus préférablement3 à 6 résidus d'un ou plusieurs acides aminés.

11. Procédé selon l'une quelconque des revendications précédentes, **caractérisé en ce que** le peptide est substitué au niveau du groupe α-amino terminal par un groupement lipophile choisi dans le groupe composé des groupements acétyle, benzoyle ou tétradécylaminocarbonyle.

12. Procédé selon l'une quelconque des revendications précédentes, **caractérisé en ce que** le peptide intermédiaire comprend au moins une arginine protégée sur la chaîne latérale par Mtr-, Pmc- ou Pbf- et/ou au moins une lysine protégée sur la chaîne latérale par Boc, l'acide 2,3-diaminopropionique ou l'acide 2,4-diaminobutyrique.

13. Procédé selon l'une quelconque des revendications précédentes, **caractérisé en ce que** le peptide intermédiaire est choisi dans le groupe composé de Bz-Gly-His(Trt)-D-Phe-Arg(Pbf)-DTrp-N(Pr)₂, Bz-Gly-His-D-Phe-Arg(Pbf)-D-Trp-N(Pr)₂, Bz-Gly-His-D-Phe-Arg(Boc)2-DTrp-N(Pr)₂, Bz-Gly-His(Trt)-D-Phe-Arg(Boc)2-D-Trp-N(Pr)₂, TDAC-Dab(Boc)-Val-Dab(Boc)-OH, TDAC-Dab(Boc)-Val-Dab(Boc)-O*^{t}*Bu, TDAC-Dab(Boc)-Val-Dab(Boc)-O-resin, Ac-Lys(Boc)-Val- Lys(Boc)-Val- Lys(Boc)-Val-OH.
